# EUROPEAN PATENT APPLICATION

(11) **EP 3 231 367 A1**
(43) Date of publication of application: **18.10.2017**
(21) Application number: 15867940.7
(22) Date of filing: 11.12.2015
(51) Int. Cl.: A61B 5/053

(54) **CASE FOR ELECTRODE MODULE**

(30) Priority: 12.12.2014 BR 102014031274
(71) Applicant: Timpel S.A., 05417-020 São Paulo - SP (BR)
(72) Inventor: HOLZHACKER, Rafael, 05417-020 São Paulo - SP (BR); CANDIANI, Igor Nowaski, 05711-000 São Paulo - SP (BR)
(74) Representative: Schwabe - Sandmair - Marx
(86) International application number: PCT/BR2015/050245
(87) International publication number: WO 2016/090449

(57) **Abstract**

The present invention refers to a casing for modules of electrodes of electrical impedance tomography. The casing comprises a foundation (2) comprising: at least one accommodation portion (3) of at least one module (4) of electrodes (7); and at least one electrical contact portion (5), having at least one window (6); being the electrical contact portion (5) foldable over the accommodation portion (3) in a way that at least one window (6) is place on at least one electrode (7).

## Description

### FIELD OF THE INVENTION

The present invention refers to a casing for modules of electrodes of electrical impedance tomography.

### BACKGROUND OF THE INVENTION

The electrical impedance tomography (EIT) is an image acquisition technique based on the application of alternating electrical signals, on the surface of patient's body, with frequencies between 10 kHz and 2.5 MHz. The equipment used for this purpose has several sensors (electrodes) placed on the skin. They are connected, by means of electrical conductors, to a processing unit that makes the mentioned alternating signal. The method used comprises several steps. In each of the steps a pair of electrodes is selected for the injection of the signal mentioned, while the induced voltage is measured, which are acquired by the electrodes that were not selected. On the following steps, other pairs of electrodes are selected for the injection of signal, continuing this sequence until all electrodes from the equipment have been selected, completing one exploring cycle. The induced tensions that were acquired by the electrodes undergo a specific software treatment, allowing the image generation that usually represents the ventilation and perfusion phenomena in the organism observed.

Normally, the electrodes are held by a belt, which is placed around the body of the patient, preferably in the thorax area. A solution quite known to the market are the modular belts, in which electrodes are serially placed in modules. In this regard, it is relevant to mention the document PI 0704408-9 that describes modular belts that have multiple electrodes, to be fastened around a part of the body of a human or animal patient.

These modular belts (or simply modules) are usually made of a flexible polymeric material in order to suit the shape of a patient's thorax. However, even with the belts being made with a flexible polymeric material, its direct contact with the patient's skin may cause discomfort. Therefore, there is the need to find a solution that provides greater comfort to the patient, softening the contact of the electrode module with the skin. Besides, some models of the belt used today apply an excessive pressure in order to be fastened with no gaps to the patient's thorax. In this regard, it is perceived that there is a shortage of means that allow the fastening of the belt without applying such pressure. Still, it is clear in the state of the art that the simple contact of the electrode with the skin may not be as effective as the electrical contact. This is even more serious when the belt is tightly fastened around the thorax. In this way, there is also the need to promote means that improve the electrical contact of the electrodes with the skin.

Therefore, to solve the problems exemplified above and other existing problems in the state of the art, one of the present invention's objective is to further a casing for electrode module of electrical impedance tomography that allows the soft and comfortable contact with the patient's skin, as well as the fastening on the thorax with no excessive pressure and efficient in terms of electrical contact.

The present invention, by means of its own characteristics, can, still, solve other problems of the state of the art not brought up here for example, once the repertoire here discussed regarding electrode belts and its problems is exemplary and non-exhausting.

### DESCRIPTION OF THE INVENTION

In order to avoid the inconveniences of the state of the art mentioned above, among others, the present invention is a casing for a module of electrodes with a foundation that comprises: at least one accommodation portion of at least one electrode module; and at least one electrical contact portion, endowed with at least one window; being the electrical contact portion foldable over the accommodation portion in a way that at least one window is placed over at least one electrode.

According to the additional and/or alternative embodiment of the present invention, the following characteristics, and its possible variations, may also be present, alone or combined:
- the electrical contact portion comprises a back face and a front face, wherein the front face is superimposed on the electrode module when the electrical contact portion is folded over the accommodation portion, wherein the back face comprises and adhesive layer.
- the adhesive layers extend through the back face of the electrical contact portion, wherein a first protective skin is placed on the said layer, that has a rupture in its extension, and further wherein over at least one window; on the front face of the electrical contact portion, a second protective skin is placed.
- the adhesive layer is made of an electrical conducting material.
- the foundation is made of a flexible and biocompatible material;
- the foundation is made of non-woven fabric;
- at least one window comprises an area of at least 80% of the area of an electrode and of a module of electrodes;
- the casing comprises at least two windows, wherein the distance De between the windows is predetermined;
- the casing is of a predetermined size within a first, second, third, fourth, fifth or sixth size;
- the predetermined distance De may vary from:
   19.3 mm to 21.3 mm, for a first size module;
   23.1 mm to 25.1 mm, for a second size module;
   25.9 mm to 27.9 mm, for a third size module;
   29.0 mm to 31.0 mm, for a fourth size module;
   32.4 mm to 34.4 mm, for a fifth size module; and
   36.2 mm to 38.2 mm, for a sixth size module;
- the foundation comprises at least one tab that is projected sideways from the accommodation portion, wherein the said tab has means of fastening that cooperate with the means of fastening of another casing's tab;
- the means of fastening comprise a "hook-loop" type mechanism;
- the foundation comprises adhesive means placed on at least one of the edges of the electrical contact portion or of the accommodation portion;
- the foundation 2 comprises a perforation 17 in line lengthwise;
- the casing comprises 16 windows; and
- the predetermined distance from the center of the first window to the center of the sixteenth window (Dpd) on a first size casing is from 300 mm to 310 mm; the predetermined distance from the center of the first window to the center of the sixteenth window (Dpd) on a second size casing is from 357 mm tom 367 mm; the predetermined distance from the center of the first window to the center of the sixteenth window (Dpd) on a third size casing is from 399 mm to 409 mm; the predetermined distance from the center of the first window to the center of the sixteenth window (Dpd) on a fourth size casing is from 445 mm and 455 mm; the predetermined distance from the center of the first window to the center of the sixteenth window (Dpd) on a fifth size casing is from 496 mm to 506 mm; the predetermined distance from the center of the first window to the center of the sixteenth window (Dpd) on a sixth size casing is from 553 mm to 563 mm.

### BRIEF DESCRIPTION OF THE DRAWINGS

The objectives, functional improvements and advantages of the present invention, will be clear to those skilled in the art from the description that follows made in regard to a preferred embodiment, which refers to the attached figures. The figures are schematic, and its dimension or proportion may not correspond to reality, once they only aim at describing the invention didactically.
FIG. 1 illustrates an exploded-view of the casing of the present invention and its layers;
FIG. 2 illustrates a top plan view of the casing of FIG. 1 and of an electrode module;
FIG. 3 illustrates the electrode module placed over the accommodation portion of the present invention's casing, showing still, in detail, the alignment of the casing's windows and the module's electrodes;
FIG. 4 illustrates the second protective skin being removed;
FIG. 5 illustrates the electrical contact portion being folded over the accommodation portion.
FIG. 6 illustrates the removal of one of the protective skins from the adhesive means, for the setting of the electrical contact portion to the accommodation portion;
FIG. 7 illustrates the electrical contact portion already attached to the accommodation portion and it also shows, in detail, the rupture that appears on the first protective skin;
FIG. 8 illustrates the removal of part of the first protective skin, from the rupture;
FIG. 9 illustrates the beginning of the attachment of the casing to a patient's body, by means of the adhesive layer uncovered by the partial removal of the first protective skin;
FIG. 10 illustrates the removal of the remaining part of the first protective skin; and
FIG. 11 illustrates the attachment of the rest of the casing on the patient's body.

### DESCRIPTION OF THE PREFERRED EMBODIMENT

The invention is now described regarding its preferred embodiments, referring to the attached figures. In the following figures and description, similar parts are marked throughout the descriptive report and figures with the same reference numbers. The figures are not necessarily in scale. Certain characteristics of the invention may be shown with exaggeration of scale or schematically, and some details of conventional elements may not be shown in order to illustrate this description in a more clear and concise way. The present invention is sensitive to embodiments carried in different ways. Specific embodiments are described in details and shown in the figures, with the understanding that the description must be considered an example of its principles, and the purpose is not to limit the invention only to what is illustrated and described in the present descriptive report. We must acknowledge that the different teachings of the embodiments discussed next may be separately employed or in any appropriate combination to provide the same results desired.

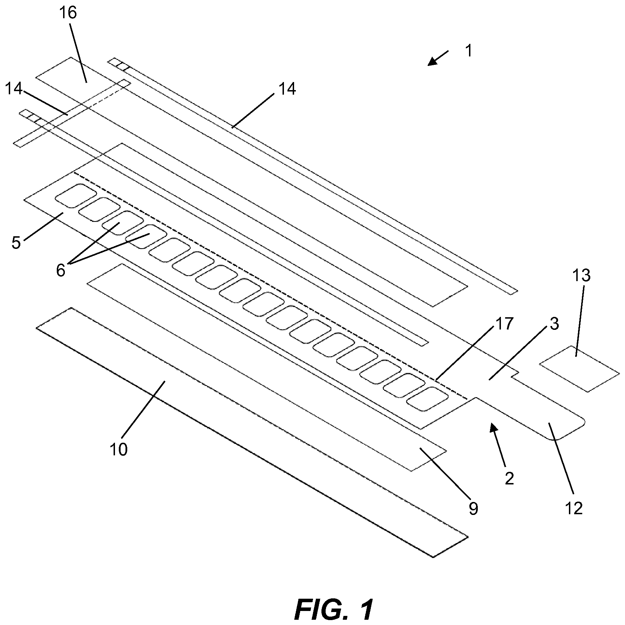

As it can be seen on FIG. 1, the casing 1 of the present invention comprises one foundation 2, which in the preferred embodiment illustrated on the figures is made of a flexible and biocompatible material, such as non-woven fabric. Evidently, other equivalent materials may be used in the making of the foundation 2. Furthermore, in particular embodiments of the present invention, the size and proportions of the casing 1 may vary according to the size of the module 4 of electrodes 7 to be enveloped, which, in its turn, should vary according to the patient that will use it. Therefore, the present invention expects the use of at least five predetermined sizes, here named as: first size, second size, third size, fourth size, fifth size and sixth size. In preferred embodiments, these sizes may have common names used in the market, such as: XS, S, M, L, XL

Still referring to FIG. 1, it is seen that the foundation 2 comprises two portions: one accommodation portion 3 of a module 4 of electrodes 7; and an electrical contact portion 5, with at least one window 6.

On the embodiment shown on the figures, the electrical contact portion 5 comprises several windows 6, wherein the distance between the windows Dej is predetermined, varying according to the size of the casing 1. To exemplify: the predetermined distance between the windows Dej on a first size casing is from 19.3 mm to 21.3 mm; the predetermined distance between the windows Dej on a second size casing is from 23.1 mm to 25.1 mm; the predetermined distance between the windows Dej on a third size casing is from 25.9 mm to 27.9 mm; the predetermined distance between the windows Dej on a fourth size casing is from 29.0 mm to 31.0 mm; the predetermined distance between the windows Dej on a fifth size casing is from 32.4 mm to 34.4 mm; the predetermined distance between the windows Dej on a sixth size casing is from 36.2 mm to 38.2 mm. Besides the distance standards, each window 6 comprises an area of at least 80% of the area of an electrode 7 of a module 4.

Still, in a preferred embodiment of the present invention, the casing 1 comprises 16 windows. Furthermore, also regarding a preferred embodiment of the present invention, the distance between the center of the first window and the center of the sixteenth window Dpd is predetermined, possibly varying according to the size of the casing 1. To exemplify: the predetermined distance from the center of the first window to the center of the sixteenth window Dpd on a first size casing is from 300 mm to 310 mm; the predetermined distance from the center of the first window to the center of the sixteenth window Dpd on a second size casing is from 357 mm tom 367 mm; the predetermined distance from the center of the first window to the center of the sixteenth window Dpd on a third size casing is from 399 mm to 409 mm; the predetermined distance from the center of the first window to the center of the sixteenth window Dpd on a fourth size casing is from 445 mm and 455 mm; the predetermined distance from the center of the first window to the center of the sixteenth window Dpd on a fifth size casing is from 496 mm to 506 mm; the predetermined distance from the center of the first window to the center of the sixteenth window Dpd on a sixth size casing is from 553 mm to 563 mm.

The electrical contact portion 5 still comprises a back face 8 over which is placed and stretched an adhesive layer 9, which, in the preferred embodiment of the figures, is made of electrical conductive material, such as a conductive solid gel. Additionally, a first protective skin 10 is placed over the adhesive layer 9, as it can be seen on FIG. 1. On the opposite side, that is, on the front face of the electrical contact portion 5, on the windows 6, a second protective skin 16 is placed.

Still referring to FIG. 1, it is noticeable that the casing 1 comprises a tab 12 that is projected sideways from the accommodation portion 3, wherein the said tab 12 has means of fastening 13 that cooperate with the means of fastening 13 of a casing 1 or of another tab 12 of a different casing 1. Therefore, in the event of two modules 4 being applied around a patient, two casings 1 will envelop each of them. In these situations, the casings 1 are united with each other by means of the tabs 12 and, more precisely, by its means of fastening 13. In a preferred embodiment of the present invention the means of fastening 13 comprise a "hook-loop" type mechanism.

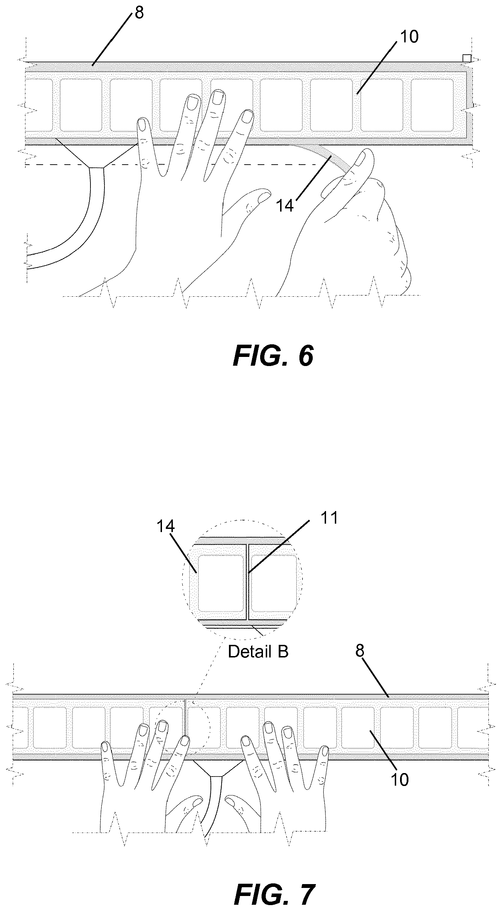

Still, the foundation 2 comprises adhesive means 14 on its edges. On the figures, the adhesive means are placed on the front face of the foundation 2, on three of its four edges. Evidently, the placement and amount of adhesive means may vary within the scope of the present invention. In the preferred embodiment, the adhesive means also comprise protective skins, as it may be seen of FIG. 6.

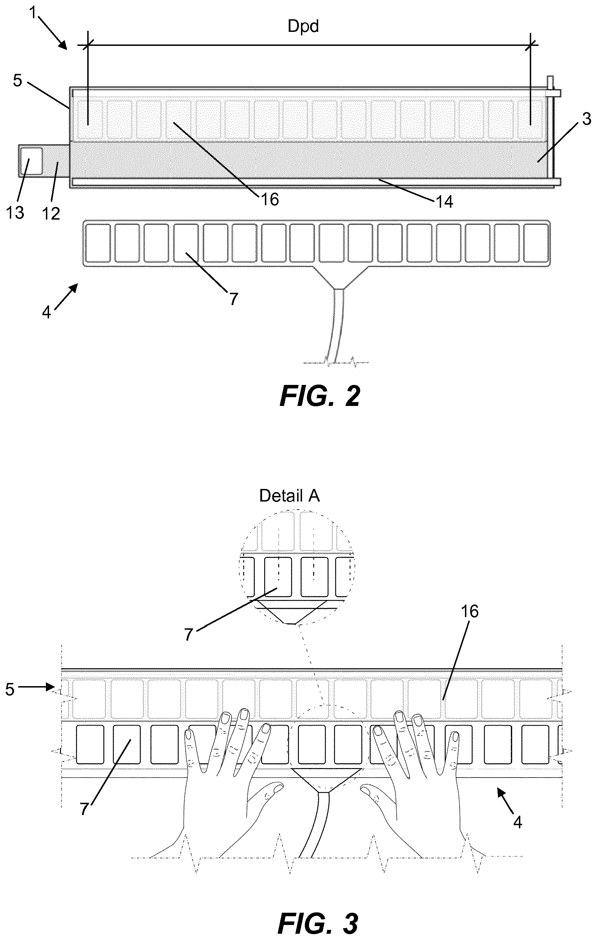

The other features of the casing 1 are described based on FIGS. 2 to 11, which show an example of application of the present invention.

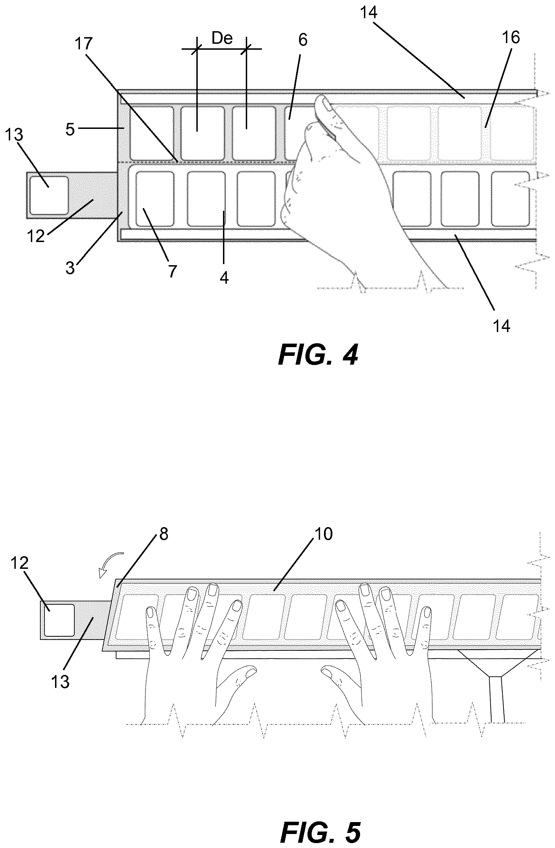

FIG. 2 shows a superior view of the casing 1, making it possible to observe the portions of accommodation 3 and electrical contact 5. As shown on FIG. 3, the module 4 of electrodes 7 is placed on the accommodation portion 3, in a way that the windows 6 are lined with the electrodes 7, such as shown on detail A. Next, as FIG. 4 shows, the second protective skin 16 is removed, exposing the adhesive layer 9, through the windows 6. FIG. 5 illustrates that the electrical contact portion 5 is foldable over the accommodation portion 3, in a way that the windows 6 are placed over the electrodes 7 and so remain aligned to them. Additionally, it is noted on FIG. 5 that the front face of the electrical contact layer 5 is superimposed to the module 4 of electrodes 7 when the electrical contact portion 5 is folded over the accommodation portion 3. Furthermore, FIG. 5 shows the first protective skin 10 on the adhesive layer 9, which is placed on the back face 8 of the electrical contact portion 5. After folding the electrical contact portion 5 over the accommodation portion 3, we must attach this portions to one another so the foundation 2 envelops the module 4 of electrodes 7. Therefore, the protective skins are removed from the adhesive means 14, and, then, portions 3 and 5 are joined, as it can be seen on FIG. 6.

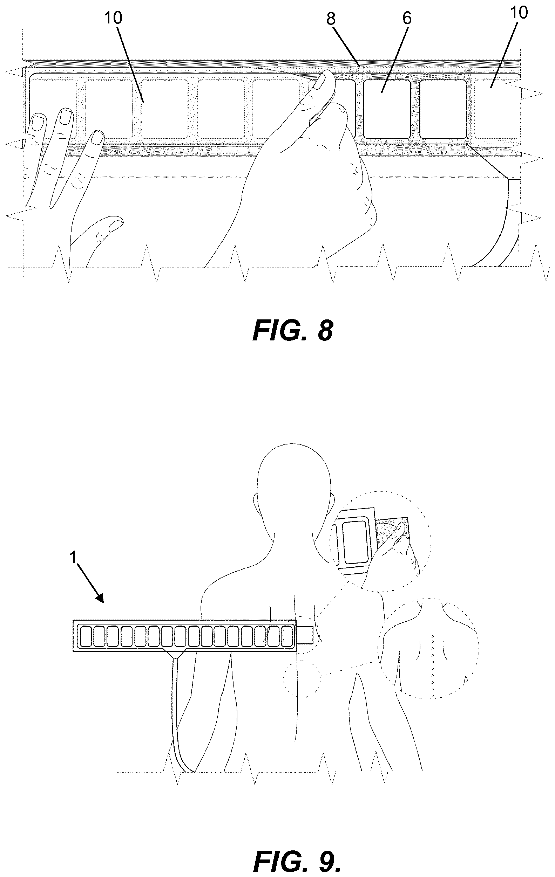

Thereafter, module 4 is enveloped by the casing 1 of the present invention, in a way that, the next step is its application on the patient's body. In this sense, it is observed that the detail B from FIG. 7 reveals a rupture 11 on the length of the first protective skin 10. The exact location of this rupture 11 may vary according to the embodiment of the invention. In exemplary terms, such rupture 11 may occur on an equivalent point at approximately 3/8 of the extension of the electrical contact portion 5, wherein, in a casing of 16 windows, six of them are located before the rupture 11 and the other 10 after it. From the rupture 11, part of the extension of the adhesive layer 9 can be uncovered and applied to the patient's skin, as FIGS. 8 and 9 show.

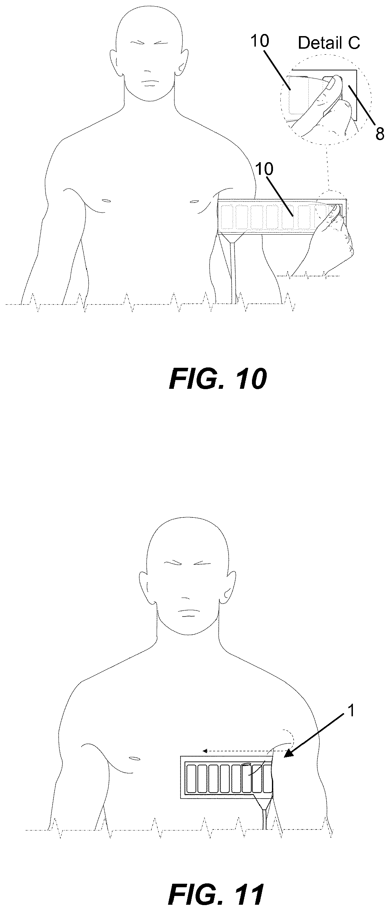

After fixing this part on the patient's skin, you can than proceed with fixing the rest of the casing 1, removing the other part of the first protective skin 10, as FIG. 10 shows, in its detail C, and applying the rest of the adhesive layer 9 on the patient's skin, as FIG. 11 shows.

The foundation 2 also comprises a perforation 17 in line lengthwise. This perforation 17 allows a quick removal of the module 4 from inside the casing 1, because it is possible to "rip" it apart to remove the module 4 from its interior.

As the description above exposes, the casing of the present invention provides, at the same time, enveloping the module 4 of electrodes 7 and a soft and comfortable contact with the patient's skin, because it is made of flexible biocompatible material.

Additionally, the adhesive layer 9 offers a considerable improvement on the contact of the skin with the electrodes 7, because it combines the adhesive fixation to the electrical conductive properties.

Still the same adhesive layer 9 allows the fixing of the casing to be done without applying excessive pressure on the patient's body, once the adhesive itself ensures a tight attachment to the skin, without pressing the patient's body such as the modular belts of the state of the art.

Another advantage that can be mentioned lies on the rupture 11, which allows the partial application of the module 4 of electrodes 7 enveloped by the casing 1 on the patient's skin, because this rupture 11 allows the adhesive parts to be exposed only when they are being attached to the patient. Unlike the total application, which needs removing the patient from the bed subjecting him/her to a certain discomfort, the partial application ensures that the patient does not have to be moved around to have the module 4 fixed on the skin. This ease can be easily perceived when dealing with a patient in a coma or seriously impaired, who cannot be put under sudden or abrupt movements, and when the module 4 is misplaced you need to completely remove it and restart the process. On the partial application, it is possible to correct the problem of a misplacement of the casing 1 more easily.

Despite the casing being specially useful to envelop modules of electrodes, the present invention can be implemented in other ways of applications and may present modifications in the manner by which it is implemented, so that the scope of protection of the invention is so only limited by the content of the following claims, including there the possible equivalent variations.

## Claims

1. CASING OF MODULES OF ELECTRODES, wherein it comprises a foundation (2) comprising: at least one accommodation portion (3) of at least one module (4) of electrodes (7); and at least one electrical contact portion (5), having at least one window (6); being the electrical contact portion (5) foldable over the accommodation portion (3) in a way that at least one window (6) is place on at least one electrode (7).

2. CASING of claim 1, wherein the electrical contact portion (5) comprises a back face (8) and a front face, being that the front face superimposes the module (4) of electrodes (7) when the electrical contact portion (5) is folded over the accommodation portion (3) and wherein the back face (8) comprises an adhesive layer (9).

3. CASING of claim 2, wherein the adhesive layer (9) extends through the back face (8) of the electrical contact portion (5), wherein a first protective skin (10) is placed on the said layer (9), which has a rupture (11) in its extension and further wherein over at least one window (6), on the front face of the electrical contact portion (5), a second protective skin (16) is placed.

4. CASING of claim 2, wherein the adhesive layer (9) is made of electrical conductive material.

5. CASING of claim 1, wherein the foundation (2) is made of flexible biocompatible material.

6. CASING of claim 5, wherein the foundation (2) is made of non-woven fabric.

7. CASING of claim 1, wherein at least one window (6) comprises an area of at least 80% of the area of an electrode (7) of a module (4) of electrode (7).

8. CASING of claim 1, wherein it comprises at least two windows (6), wherein the distance between the windows (Dej) is predetermined.

9. CASING of claim 1, wherein each one is of a predetermined size within a first, second, third, fourth, fifth or sixth size.

10. CASING of claims 8 and 9, wherein the predetermined distance (Dej) may vary from:
19.3 mm to 21.3 mm, for a first size casing;
23.1 mm to 25.1 mm, for a second size casing;
25.9 mm to 27.9 mm, for a third size casing;
29.0 mm to 31.0 mm, for a fourth size casing;
32.4 mm to 34.4 mm, for a fifth size casing; and
36.2 mm to 38.2 mm, for a sixth size casing;

11. CASING of claim 1, wherein the foundation (2) comprises at least one tab (12) that is projected sideways from the accommodation portion (3), wherein the said tab (12) has means of fastening (13) that cooperate with means of fastening (13) of another casing (1).

12. CASING of claim 11, wherein the means of fastening (13) comprise a "hook-loop" type mechanism.

13. CASING of claim 1, wherein the foundation (2) comprises adhesive means (14) placed on at least one of the edges of the electrical contact portion (5) or of the the accommodation portion (3).

14. CASING of claim 1, wherein the foundation (2) comprises a perforation (17) in line along its extension.

15. CASING of claim 1, wherein it comprises 16 windows (6).

16. CASING of claim 15, wherein the predetermined distance from the center of the first window to the center of the sixteenth window (Dpd) on a first size casing is from 300 mm to 310 mm; the predetermined distance from the center of the first window to the center of the sixteenth window (Dpd) on a second size casing is from 357 mm tom 367 mm; the predetermined distance from the center of the first window to the center of the sixteenth window (Dpd) on a third size casing is from 399 mm to 409 mm; the predetermined distance from the center of the first window to the center of the sixteenth window (Dpd) on a fourth size casing is from 445 mm and 455 mm; the predetermined distance from the center of the first window to the center of the sixteenth window (Dpd) on a fifth size casing is from 496 mm to 506 mm; the predetermined distance from the center of the first window to the center of the sixteenth window (Dpd) on a sixth size casing is from 553 mm to 563 mm.
